# EUROPEAN PATENT APPLICATION

(11) **EP 2 168 541 A1**
(43) Date of publication of application: **31.03.2010**
(21) Application number: 08165184.6
(22) Date of filing: 25.09.2008
(51) Int. Cl.: A61F 13/15

(54) **A re-usable undergarment comprising a modified crotch portion**

(71) Applicant: MKS-DEVO Tekstil ve Kimyevi Maddeler Sanayi Ve Ticaret A.S., Istanbul (TR)
(72) Inventor: Sengün, Mehmet Korgun MKS & Devo Tekstil ve Kimyevi Maddeler San.Tic. AS, Istanbul (TR)
(74) Representative: Sevinç, Erkan

(57) **Abstract**

A re-usable undergarment which comprising a body portion and a crotch portion and which is free of superabsorbents is disclosed. The crotch portion has at least three air-permeable layers attached to each other from their side edges so as to create empty volumes in between the attachment lines of each of the layers. The first layer is on the wearer's side and has an upper surface and a lower surface, the lower surface being made hydrophobic and oleophobic by application of a fluorocarbon based chemical paste, whereas the former is hydrophilic. The second layer is located underside the first layer and has an upper surface and a lower surface, one of which is made hydrophobic and oleophobic. The third layer is located underside the second layer and has an upper surface and a lower surface, the upper surface is made hydrophobic and oleophobic.

## Description

### Technical Field of the Invention

The present invention relates to undergarments and more particularly to a re-usable undergarment which is air permeable and has antibacterial characteristics with a modified crotch portion for retention of body fluids and prevention of leakage.

### Background of the Invention

Natural fibers as cotton, rayon, viscose and bamboo are the most known and preferred fibers for the manufacture of undergarment fabrics. These fabrics are liquid permeable and oleophilic so that undergarments manufactured from these fabrics swallow the body fluids and retains some of these fluids on the top and/or inner side while transmitting some to the outer side of the undergarment surface. The retained fluids cause the person to feel wetness and the transferred fluids soils to the clothes of the wearer. This situation causes production of bacteria and other microorganisms, formation of an unhealthy medium and an uncomfortable feeling for the skin of the wearer.

For the purpose of improving dryness and comfortable feeling, the prior art teaches some modifications on the surface of the fabrics. As an example, US 2004/037963 discloses a process for manufacturing a textile surface with hydrophobic properties on the one side and hydrophilic properties on the other side by the application of a paste that comprises an emulsion or dispersion of paraffin, polysiloxane and/or fluorine compounds to the surface of textile material. Application of these chemicals onto a textile surface makes the textile material liquid permeable from one side to its other side.

EP 0 985 392 discloses a disposable absorbent article having at least three layers and one super hydrophobic surface which is coated by means of modulated plasma deposition of fluorocarbons. In addition, the absorbent article includes a fluid storage layer that is positioned in fluid communication with, and underlying a primary or a secondary distribution layer and comprises an absorbent gelling material like hydrogels, superabsorbent or hydrocolloid materials.

Another absorbent article is disclosed in GB 2 409 647 where a sanitary undergarment that includes an absorbent pad incorporated into the gusset to prevent slippage is proposed. The undergarment is designated for single use and entirely disposable.

Present invention discloses a re-usable undergarment which comprises an improved crotch portion and is suitable both for daily use and for female sanitation use. The crotch portion has at least three layers whose top and back surfaces have different characteristics such as water permeable/impermeable and oleophobic/oleophilic. The liquids, especially body fluids, can be easily removed from the first layer of the crotch portion that is in contact with the skin of the wearer and the second layer of the crotch portion does not permit the liquids to leak and to soil to the clothes of the wearer.

### Objects of the Invention

An object of the present invention is to provide an undergarment whose modified crotch region allows removal of body fluids from the top layer into a volume between inner layers where said body fluids are trapped.

Another object of the present invention is to provide an undergarment which is re-usable and suitable both for daily use as well as during female sanitation.

Another object of the present invention is to provide an undergarment that promotes hygiene by keeping the crotch area dry and by using an improved fabric that has antibacterial and breathable characteristics.

Yet another object of the present invention is to provide an undergarment which does not require, for containing body fluids, an additional material such as an absorbent pad for daily use and also during female sanitation.

### Summary of the Invention

The objects of the present invention are achieved through use of an improved fabric for the manufacture the undergarment that preferably has three layered and privately designed crotch portion.

The fabric that is manufactured from natural fibers is coated with a mixture of chemicals such as fluoroalky polymers, isocyanate, polyether polyalcohol, polysiloxane to obtain a surface having hydrophobic character.

In a preferred embodiment of the present invention the crotch portion of the undergarment is designed as to be three layered where each layer has one surface which is both hydrophobic and oleophobic. The crotch portion absorbs the body fluids and stores the same in volumes between these layers. Stored fluids can be removed by washing the undergarment after usage if they do not disappear by evaporation.

The re-usable undergarment comprises a body portion and a crotch portion and is free of superabsorbents. The crotch portion has at least three air-permeable layers attached to each other from their side edges so as to create empty volumes in between the attachment lines of each of the layers. The first layer is on the wearer's side and has an upper surface and a lower surface, the latter being made hydrophobic and oleophobic by application of the aforementioned chemical paste, whereas the former is hydrophilic. The second layer is located underside the first layer and has an upper surface and a lower surface, one of which is made hydrophobic and oleophobic by application the chemical paste and the other one is hydrophilic. Likewise, the third layer is located underside the second layer and has an upper surface and a lower surface, the upper surface is made hydrophobic and oleophobic.

### Brief Description of the Figures

Figure 1 shows the unsewed view of the undergarment;
Figure 2 shows the cross-sectional view of the undergarment;
Figure 3a and 3b respectively show cross-sectional views of a hydrophobic and a hydrophilic surface;
Figure 3c shows the cross-sectional view of a layer;
Figure 4 shows the cross-sectional view of the layers of the crotch portion of a preferred embodiment of the invention;
Figure 5 shows the cross-sectional view of the layers of the crotch portion of a second preferred embodiment of the invention.

### Detailed Description of the Invention

The present invention relates to an undergarment (1) comprising of a body portion (5) and an improved crotch portion (2) that is suitable both for daily use and during female sanitation.

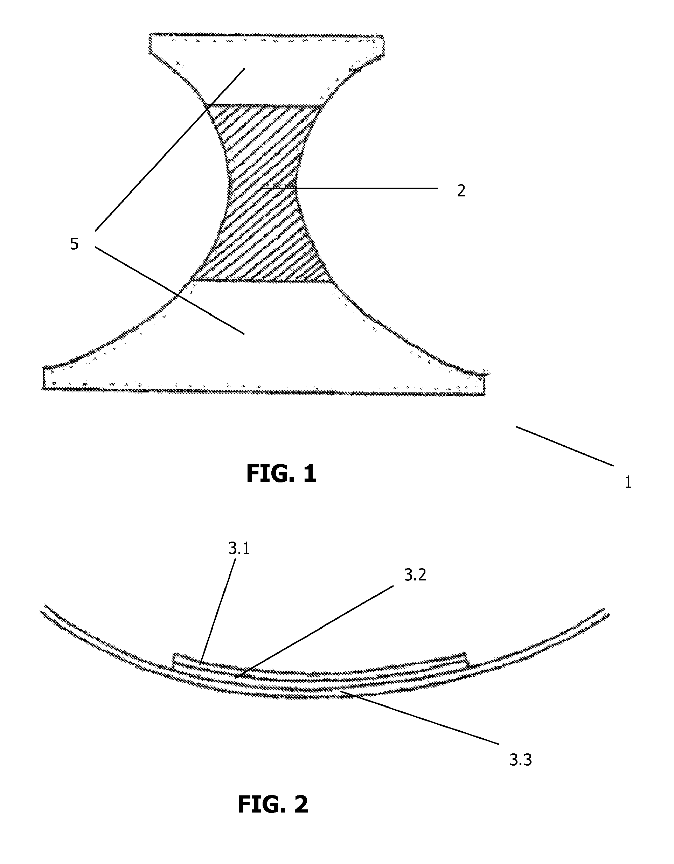

According to the present invention, the crotch portion (2) includes three layers (3) that are sewed to each other over their side edges. An empty space is formed in between the first and second layers as well as the second and the third layers. These two spaces are used to contain the body fluids penetrated from the first layers towards the inner second and third layers. Referring to Figure 2, these layers (3) are named as first layer (3.1), second layer (3.2) and third layer (3.3) from top to bottom. A chemical paste is applied to one of the surfaces (4) of each of these three layers (3). Each of the surfaces (4) coated with the paste become hydrophobic while the other faces of the layers remain hydrophilic due to the natural characteristic of the fabric. Application of the chemical paste modifies physical characteristics of the concerned surface and will therefore be referred to as modification of a surface in the remainder of this text.

The paste used for the modification of the fabric surface (4) comprises preferably around 20 wt. % of a floroalkyl polymer, around 10 wt. % of a polyether polyalcohol and/or diisocyanate, around 2 wt. % of a silicon softener (polysiloxane), around 2,5 wt. % of a ethyl acyrlate based synthetic thickener, around 0,5 wt. % of an ammonia solution with %25 concentration and around 65 wt. % of a deionized or soft water.

To obtain the paste which distinguishes hydrophobic and oleophobic characteristics of the fabric, a high speed mixer that mixes the above mentioned chemicals with a velocity of 2000 to 3000 rpm is used and the mixing process is takes place until the viscosity of the mixture is raised to 90 to 150 mPa.s. The viscose mixture is partially perforated to one surface (4) of the textile by using usually a Rotary Printing Machine that has a mesh surface with number 40 to 150. The processing velocity of the textile in the said printing machine is calculated as 5 to 10 m/min according to the viscosity of the applied mixture. In order to obtain a hydrophobic and oleophobic characterized fabric surface the paste is coated in an amount of 25 to 100 g paste per unit area of fabric whose bulk is chosen preferably between 120 to 240 g/m².

The coated fabric surface is dried in a dryer furnace or stenter at around 100°C for 1 to 2 minutes and then kept at 160-170°C for 1 to 4 minutes so as to allow the chemicals properly bind to the fabric surface. While one surface of the fabric is modified to have hydrophobic and oleophobic properties the other surface has hydrophilic and oleophilic properties as a result of the nature of the fabric. On the other hand both of the surfaces (4.1 and 4.2) are air permeable. Air permeability on the modified surface (4.2) is maintained by the micro pores on the coating that are formed during the drying process whereas the unmodified surface (4.1) has this feature in its nature.

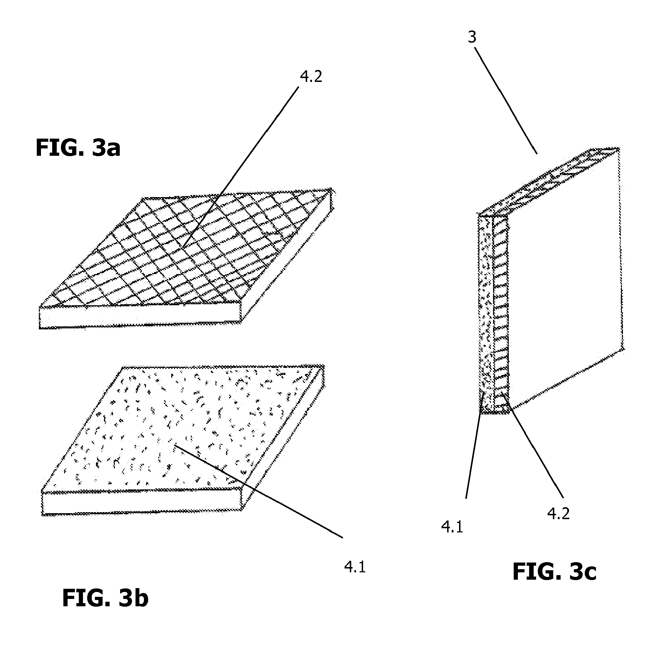

Figure 3a shows the modified surface (4.2) having hydrophobic and oleophobic properties and is air permeable. Figure 3b shows the unmodified surface (4.1) which naturally has hydrophilic, oleophilic and air permeability characteristics.
Figure 3c shows the cross-sectional view of the fabric which is used for the manufacture of the crotch portion (2) of the undergarment (1) which is a combination of the modified and unmodified surfaces (4.2 and 4.1). Both of the modified and unmodified surfaces (4.2 and 4.1) of the fabric are soft feeling and nonirritating to the wearer's skin.

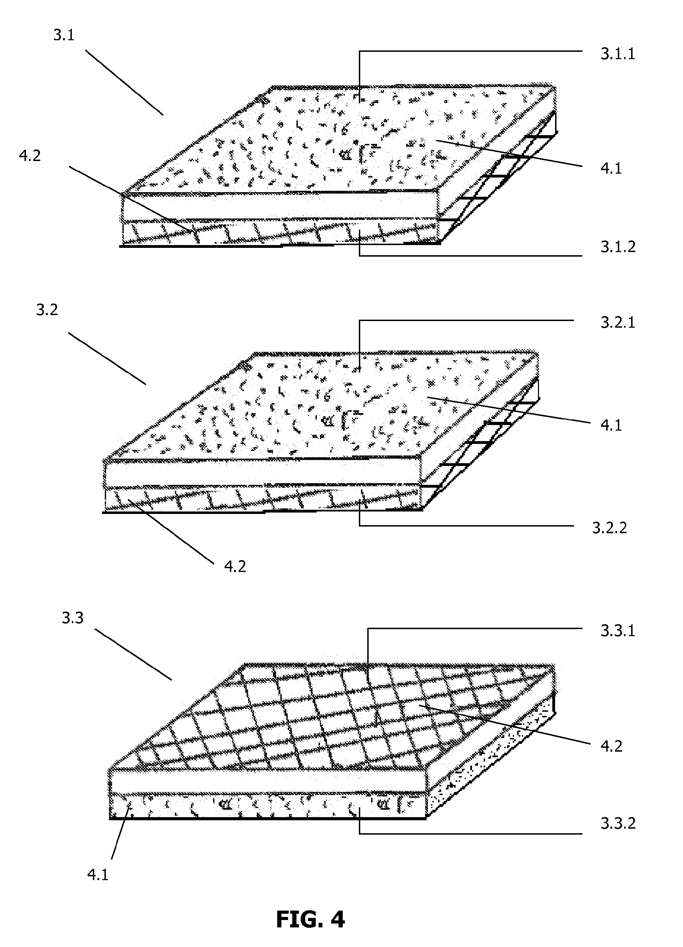

According to the first preferred embodiment of the present invention, the crotch portion (2) comprises three layers (3.1, 3.2, and 3.3). With reference to Fig. 4, the first layer (3.1) is positioned on the top, i.e. in contact with the wearer's skin. The top surface (3.1.1) of the first layer (3.1) is unmodified surface (4.1) whereas its lower surface (3.1.2) is the modified surface (4.2). The second layer (3.2) is positioned underside the first layer (3.1). The top surface (3.2.1) of the second layer (3.2) is the unmodified surface (4.1) whereas its lower surface (3.2.2) is the modified surface (4.2). The third payer (3.3) is positioned underside the second layer (3.2) and is intended to constitute outermost part of the undergarment. Unlike the first and second layers, the top surface (3.3.1) of the third layer (3.3) is the modified surface (4.2) whereas its lower surface (3.3.2) is the unmodified surface (4.1). The lower surface (3.3.2) of the third layer (3.3) is the one that is actually seen as forming the outer surface of the crotch portion.

According to this configuration, the first layer (3.1) whose top surface (3.1.1) has the hydrophilic and oleophilic characteristics serves to the purpose of absorbing the body fluids from the skin of the wearer by transferring it into the volume formed between the first and second layers of the crotch portion (2). Consequently the wetness of the top layer of the crotch portion (2) is averted and the fluids are transferred to the primary volume (not shown) between the back surface of the first layer (3.1.2) and top surface of the middle layer (3.2.1). As a consequence of the coating process, back surface of the first layer (3.1.2) has hydrophobic and oleophobic characteristic and prevents the fluids to be transferred back to the top surface of the first layer (3.1.1) and as the top surface of the middle layer (3.2.1) has hydrophilic and oleophilic characteristics, it has tendency of swallowing and transferring the fluids to the secondary volume (not shown) between the second and third layers (3.2 and 3.3). As the modified surfaces (3.2.2, 3.3.1) of the second and the third layers (3.2 and 3.3) both have the hydrophobic and oleophobic characteristics, the transferred fluid from the upper portions is conserved in the secondary volume in between the back surface (3.2.2) of the middle layer (3.2) and top surface (3.3.1) of the third layer (3.3) and because the feature of impermeability of the surfaces (3.2.2 and 3.3.1), swallowed body fluids are prevented from penetrating to other layers. In addition, all of these layer surfaces (3.1.1, 3.1.2, 3.2.1, 3.2.2, 3.3.1 and 3.3.2) have air permeability (breathability) feature that helps the removal of the moisture which comes into existence in the crotch portion (2) of the undergarment (1), as well as promoting hygiene by keeping the crotch area dry.

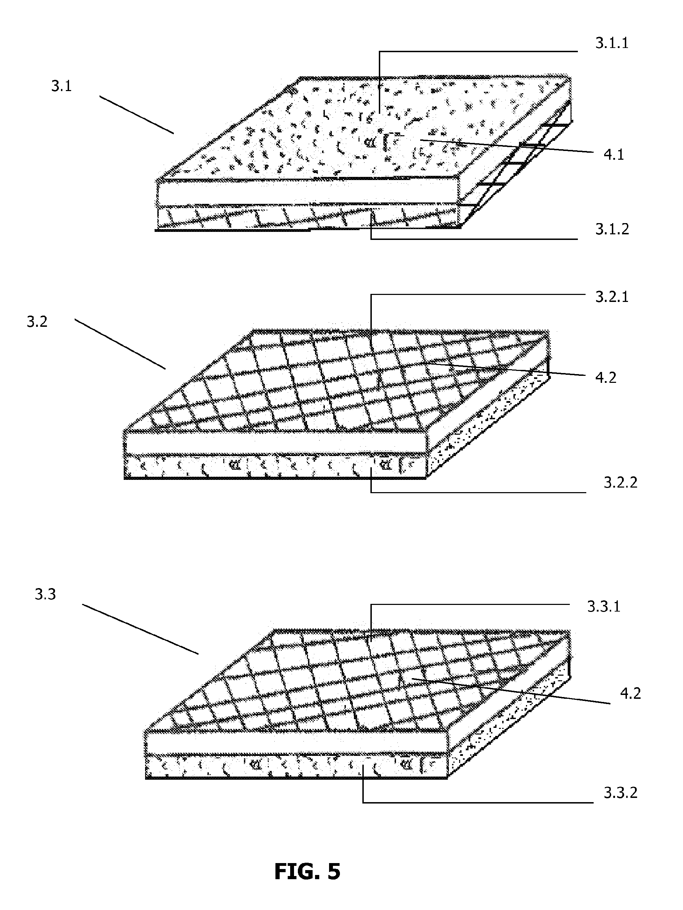

According to the second preferred embodiment of the present invention, the crotch portion (2) comprises three layers (3.1, 3.2, and 3.3). With reference to Fig. 5, the first layer (3.1) is positioned on the top, i.e. in contact with the wearer's skin. The top surface (3.1.1) of the first layer (3.1) is unmodified surface (4.1) whereas its lower surface (3.1.2) is the modified surface (4.2). The second layer (3.2) is positioned underside the first layer (3.1). The top surface (3.2.1) of the second layer (3.2) is the modified surface (4.2) whereas its lower surface (3.2.2) is the unmodified surface (4.1). The third layer (3.3) is positioned underside the second layer (3.2) and is intended to constitute outermost part of the undergarment. Unlike the first layer (3.1), the top surface (3.3.1) of the third layer (3.3) is the modified surface (4.2) whereas its lower surface (3.3.2) is the unmodified surface (4.1). The lower surface (3.3.2) of the third layer (3.3) is the one that is actually seen as forming the outer surface of the crotch portion.

According to this second configuration, the first layer (3.1) whose top surface (3.1.1) has the hydrophilic and oleophilic characteristics serves to the purpose of absorbing the body fluids from the skin of the wearer by transferring it into the volume formed between the first and second layers of the crotch portion (2). Consequently the wetness of the top layer of the crotch portion (2) is averted and the fluids are transferred to the primary volume (not shown) between the back surface of the first layer (3.1.2) and top surface of the second layer (3.2.1). As a consequence of the coating process, back surface of the first layer (3.1.2) has hydrophobic and oleophobic characteristic and prevents the fluids to be transferred back to the top surface of the first layer (3.1.1) and as the top surface of the middle layer (3.2.1) has hydrophobic to a certain extent, it has considerably less tendency of swallowing and transferring the fluids to the secondary volume (not shown) between the second and third layers (3.2 and 3.3). Still, it has been observed that some amount of fluids penetrate the second layer and pass to the secondary volume in between the second and third layers by effect of pressure, typically arising from the body weight and movements of the wearer. As the modified surfaces (3.2.1, 3.3.1) of the second and the third layers (3.2 and 3.3) both have the hydrophobic and oleophobic characteristics, the transferred fluid from the upper portions is conserved in the secondary volume in between the back surface (3.2.2) of the second layer (3.2) and top surface (3.3.1) of the third layer (3.3). Because of the impermeability feature of the surfaces (3.2.1 and 3.3.1), swallowed body fluids are prevented from penetrating to other layers or to the outside of the undergarment. In addition, all of these layer surfaces (3.1.1, 3.1.2, 3.2.1, 3.2.2, 3.3.1 and 3.3.2) have air permeability (breathability) feature that helps the removal of the moisture which comes into existence in the crotch portion (2) of the undergarment (1), as well as promoting hygiene by keeping the crotch area dry.

In the third embodiment of the invention, the layers (3) of the crotch portion (2) are coated with the fluorocarbon based paste in different proportions. As an example bamboo fabric whose bulk is 160 g/m² is coated with three different proportions of paste and the pressure threshold in respect of liquid migration from the coated surface of the fabric into the uncoated surface are compared considering the intrusion pressure applied onto the fabric according to the amount of paste coated per unit area. Hydrostatic water pressure test is carried out through these coated surfaces and a water intrusion test equipment with a water pressure rate of 60 cm H₂O/min is used for the experiment. The measurements have been carried out according to the standards BS EN 20811 and AATCC 127. It was observed that the intrusion pressure of the coated surface increases with the increased amount of coated paste per unit area. Table 1 shows the intrusion pressure of the fabric with respect to the amount of chemical paste applied onto the surface of the fabric.

| Table -1 | |
|---|---|
| Coated paste (g/m²) | Applied pressure (mbar) |
| 30 - 35 | 5-6 |
| 45 - 55 | 6-8 |
| 60-70 | 10-12 |

According to the third embodiment, the crotch portion (2) is positioned as the configuration given in figure 4 with the exception that the amount of surface modification was made different in between the layers (3.1, 3.2 and 3.3). Hydrophobic surfaces were made to have different liquid intrusion pressures in an example. Back surface of the first layer (3.1.2) of this example was coated with 30 g/m² fluorocarbon based paste whereas the back surface of second layer (3.2.2) was coated in an amount of 45 g/m² fluorocarbon based paste and whereas the top surface of last layer (3.3.1) was coated in an amount of 60 g/m² fluorocarbon based paste. These modified surfaces (4.2) does not permit the fluids to be transferred to the opposite surfaces (4.1) of the layers until the liquid pressure that is applied on the surface (4.2) is larger than the known amounts shown in Table 1. Unsurprisingly, the third layer's top surface (3.3.1) has the highest liquid intrusion pressure and the back surface of the middle layer's (3.2.2) intrusion pressure is higher than that of the first layer's surface (3.1.2). Thus, when the body fluids penetrate into the top surface of the first layer (3.1.1) and pass into the first volume between the first and second layers, much of the body fluids penetrate the second layer (3.2) and pass to the second volume in between the second and third layers. When the body liquids which still remain in the first volume exert pressure less than 5 mbar, the back surface of the first layer (3.1.2) does not permit the fluids to penetrate the top surface of the first layer (3.1.1). It was noted that when the fluids contained in the secondary volume exerts pressure less than 10 mbar onto the surfaces (3.2.2 and 3.3.1), the top surface of the third layer (3.3.1) does not permit the fluids to be penetrate the third layer and spill out of the garment.. The coating amount of the back surface of the middle layer (3.2.2) is designed as to calibrate the fluid amounts in the first and second volumes such as when the amount of the body fluid is too much in the secondary volume, the back surface of the second layer (3.2.2) automatically starts to transfer the excess fluids back to the first volume to fix the pressure of the secondary volume lower than 10 mbar, which is a threshold that should not be exceeded in order to prevent spillage out of the garment.

According to the present invention, the undergarment (1) is manufactured from natural fibers such as cotton, rayon, viscose and bamboo. As the modified surfaces (4.2) are elastic and soft likewise unmodified surfaces (4.1), the body portion (5) and the crotch portion (2) have the elasticity and softness features for the comfort of the wearer. In addition, the undergarment (1) does not produce unwanted sounds with the movement of the wearer as opposed to several diapers or women's pads made of polymeric materials.

The undergarment (1) is designed to absorb the body fluids and to be suitable for daily use and/or for women having their menstrual flow without any additional requirement to an absorbent pad. While this undergarment (1) is compatible for women, it may be manufactured as to be suitable for men or children as well.

The undergarment (1) can easily be cleaned by just washing with ample water. Once the undergarment is dried, it should be ironed to retrieve the hydrophobic and oleophobic characteristics on modified surfaces of the layers. As opposed to most of the solutions for menstruation garments of women, the undergarment of the present invention can be used as long as the modified surfaces are able to retrieve their hydrophobicity. This function can easily be controlled by changing the type and amount of binder's in the chemical paste coated on the modified surfaces. Since this is a well known issue in the textile chemistry no more detail will be given here.

## Claims

1. A re-usable undergarment (1) suitable for daily use and also for menstruation period use of women, said undergarment comprises a body portion (5) and a crotch portion (2) and is free of superabsorbents, said crotch portion (2) having a plurality of air-permeable layers attached to each other from their side edges so as to create empty volumes in between the attachment lines of each of the layers
**characterized in that** the crotch portion comprises;
a first layer (3.1) on the wearer's side and having an upper surface (3.1.1) and a lower surface (3.1.2), the latter being made hydrophobic and oleophobic by application of a fluorocarbon based chemical whereas the former (3.1.1.) is hydrophilic,
a second layer (3.2) located underside the first layer (3.1) and having an upper surface (3.2.1) and a lower surface (3.2.2), one of which being made hydrophobic and oleophobic by application of a fluorocarbon based chemical and the other one is hydrophilic, and
a third layer (3.3) located underside the second layer (3.2) and having an upper surface (3.3.1) and a lower surface (3.3.2), the former (3.3.1) being made hydrophobic and oleophobic by application of a fluorocarbon based chemical whereas the latter (3.1.1.) is hydrophilic.

2. A re-usable undergarment (1) as set forth in claim 1 wherein the liquid intrusion pressure of the third layer (3.3) is higher than the liquid intrusion pressure of the second layer (3.2).

3. A re-usable undergarment (1) as set forth in claim 1 wherein the liquid intrusion pressure of the second layer (3.2) is lower than that of the third layer (3.3.) and is higher than that of the first layer (3.1) so as to allow back penetration of fluids contained in a secondary volume formed in between the second and third layers (3.2 and 3.3) into a primary volume formed in between the first and second layers (3.1 and 3.2).

4. A re-usable undergarment according to claim 1 wherein the fabric of the body portion and the crotch portion is manufactured from one of the natural fibers cotton, rayon, viscose or bamboo.

5. A re-usable undergarment according to claim 1 wherein the fluorocarbon based chemical paste is a mixture fluoroalkyl polymers, isocyanate, polyether polyol and polysiloxane.

6. A re-usable undergarment according to claim 1 wherein the fluorocarbon based chemical paste contains 20 wt. % of a floroalkyl polymer, 10 wt. % of a polyether polyalcohol and/or diisocyanate, 2 wt. % of a silicon softener (polysiloxane), 2,5 wt. % of a ethyl acyrlate based synthetic thickener, 0,5 wt. % of an ammonia solution with %25 concentration and 65 wt. % of a deionized or soft water.
